(19) Europäisches Patentamt
European Patent Office
Office européen des brevets

(11) Veröffentlichungsnummer : **0 115 325 B1**

# (12) EUROPÄISCHE PATENTSCHRIFT

(45) Veröffentlichungstag der Patentschrift : **29.07.87**

(51) Int. Cl.⁴ : **C 07 D239/50**, C 07 D239/48

(21) Anmeldenummer : **84100718.0**

(22) Anmeldetag : **24.01.84**

## (54) Verfahren zur Herstellung von 2-substituierten 5-Nitroso-4,6-diamino-pyrimidinen.

(30) Priorität : **28.01.83 CH 482/83**

(43) Veröffentlichungstag der Anmeldung : **08.08.84 Patentblatt 84/32**

(45) Bekanntmachung des Hinweises auf die Patenterteilung : **29.07.87 Patentblatt 87/31**

(84) Benannte Vertragsstaaten :
**BE CH DE FR GB IT LI LU NL**

(56) Entgegenhaltungen :
**FR-A- 1 364 734**
**FR-A- 2 370 738**
**CHEMICAL ABSTRACTS, Band 47, 1953, Spalte 5946e,f, Columbus, Ohio, USA H. SATO et al.: "Pteridines. III. The synthesis of antagonists to folic acid"**
**JOURNAL OF THE AMERICAN CHEMICAL SOCIETY, Band 69, Juli 1947, Seiten 1814-1816, Washington, USA M.F. MALLETTE et al.: "Pyrimido (4,5-b) pyrazines. II. 2,4-Diaminopyrimido (4,5-b) pyrazin e and derivatives"**
**JOURNAL OF THE AMERICAN CHEMICAL SOCIETY, Band 81, 20. Mai 1959, Seiten 2442-2448, Washington, USA E.C. TAYLOR et al.: "Studies in purine chemistry. II. A facile synthesis of 2-substituted adenines"**

(73) Patentinhaber : **LONZA AG**
**Gampel/Wallis (CH)**

(72) Erfinder : **O'Murchu, Colm, Dr.**
**Gebreitenweg 4 B**
**Visp (Kanton Wallis) (CH)**

(74) Vertreter : **Weinhold, Peter, Dr. et al**
**Patentanwälte Dr. V. Schmied-Kowarzik Dipl.-Ing. G. Dannenberg Dr. P. Weinhold Dr. D. Gudel Dipl.-Ing. S. Schubert Dr. P. Barz Siegfriedstrasse 8**
**D-8000 München 40 (DE)**

**EP 0 115 325 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Es ist bekannt, dass sich das 5-Nitroso-2,4,6-triamino-pyrimidin aus Malonsäuredinitril und einem Guanidinsalz herstellen lässt.

Durch Kondensation von Malonsäuredinitril und Guanidin-hydrochlorid bzw. -nitrat in Gegenwart von Natriumalkoholat in alkoholischer Lösung erhält man 2,4,6-Triamino-pyrimidin in mässiger Ausbeute [W. Traube, Ber. 37, 4544 (1904) ; H. Sato et al. J. Chem. Soc. Japan Pure Chem. Sect. 72, 866 (1951), Chem. Abstr. 47, 5946 (1953)]. Dieses Pyrimidin wird dann zu 5-Nitroso-2,4,6-triamino-pyrimidin mit salpetriger Säure nitrosiert [M.F. Mallette et al, J. Am. Chem. Soc. 69, 1814 (1947)]. Diese Verfahren haben den Nachteil, dass sie zu umständlich sind für die Herstellung von grösseren Mengen, und dass die Ausbeute an 5-Nitroso-2,4,6-triamino-pyrimidin höchstens 75 bis 78 %, bezogen auf Malonsäuredinitril, beträgt.

Es wurde versucht, dieses Verfahren zu vereinfachen, indem man das Zwinschenprodukt 2,4,6-Triamino-pyrimidin nicht isolierte (CH-PS 630 616). Diesem Verfahren haften aber immer noch verschiedene Nachteile an, es muss teures Natriumalkoholat verwendet werden, pro Mol eingesetztes Malonsäuredinitril fallen mindestens zwei Mol Salz (NaCl und Na-acetat) an, es muss mit relativ verdünnten Reaktionslösungen gearbeitet werden (ca. 2 l Lösungsmittel pro Mol Produkt), und schliesslich gestaltet sich die Wiederaufarbeitung der Lösungsmittel als sehr schwierig, da es sich um ein 4-Komponenten-Lösungsmittelgemisch (Methanol, Aethanol, Eisessig, Wasser und Nebenprodukte) handelt.

Ein anderer Weg, 5-Nitroso-2,4,6-triamino-pyrimidin herzustellen, ist in E.C. Taylor, O. Vogl and C.C. Cheng, J. Am. Chem. Soc. 81, 2442 (1959) beschrieben. Durch Erhitzen des Kaliumsalzes von Isonitroso-malonitril mit Guanidin-carbonat in Dimethylformamid erhält man 5-Nitroso-2,4,6-triamino-pyrimidin in 88 % Ausbeute. Da das Kaliumsalz aus dem Silbersalz von Isonitroso-malonitril hergestellt wird, kommt dieses Verfahren für eine grosstechnische Produktion nicht in Frage.

In der FR-PS 1 364 734 wird ein Verfahren beschrieben, in dem zuerst das Malonsäuredinitril in wässriger Essigsäure-Lösung mit Natriumnitrit nitrosiert, dann die gebildete Isonitrosomalonitril-Lösung mit Guanidin-carbonat behandelt wird, wobei $CO_2$ entweicht und das Guanidinsalz von Isonitroso-malonitril ausfällt. Diese Salz-Suspension wird dann auf ca. 0 °C abgekühlt, filtriert und das Guanidinsalz von Isonitrosomalonitril getrocknet. Das Salz wird dann in Dimethylformamid nach Zugabe von $K_2CO_3$ unter Rückfluss erhitzt, um die Isomerisierung zu 5-Nitroso-2,4,6-triamino-pyrimidin zu vollziehen.

Dieses Verfahren stellt einen gewissen Fortschritt gegenüber dem Verfahren nach Taylor dar, weist aber noch mehrere Nachteile auf. So muss die Essigsäure im Ueberschuss verwendet werden (10 % laut Beispiel). Dieser Ueberschuss muss mit Guanidin-carbonat neutralisiert werden, um das Guanidinsalz von Isonitroso-malonitril vollständig zu bilden.

Das Kühlen von wässriger Suspension auf ca. 0 °C ist technisch mit Schwierigkeiten verbunden, weil eine Kruste von Eis an der inneren Seite des Reaktionsgefässes gebildet wird. Beim Kühlen auf weniger tiefe Temperaturen wird das Salz nicht vollständig ausgefällt.

Das Trocknen des Guanidinsalzes von Isonitroso-malonitril ist aus sicherheitstechnischen Gründen riskant.

Bei der Behandlung von roher Isonitroso-malonitril-Lösung mit Guanidin-carbonat wird ein Aequivalent $CO_2$ freigesetzt. Das Reaktionsgemisch hat daher die Neigung, während dieses Arbeitsganges zu schäumen, so dass das Reaktionsgefäss nicht optimal ausgenutzt werden kann.

Aufgabe der vorliegenden Erfindung war es, diese Nachteile zu vermeiden und ein Verfahren vorzuschlagen, das es ermöglicht, auf einfache und wirtschaftliche Weise und in hoher Ausbeute 2-substituierte 5-Nitroso-4,6-diamino-pyrimidine herzustellen.

Erfindungsgemäss wurde das mit einem Verfahren nach Anspruch 1 erreicht.

Das Malonsäuredinitril wird nach dem Verfahren der Erfindung in Wasser oder Alkohol als Lösungsmittel mit einem Amidin oder Guanidin in saurem Milieu in Gegenwart eines Nitritsalzes nitrosiert, wobei sich das Amidin- bzw. Guanidinsalz von Isonitroso-malonitril direkt in wässriger oder alkoholischer Suspension bildet. Dieses Salz wird nicht isoliert, sondern das Gemisch wird basisch gestellt und nach Zugabe von Dimethylformamid oder einer Pyridinbase wird das Wasser oder der Alkohol unter vermindertem Druck abdestilliert. Nachdem das Wasser oder der Alkohol fast vollständig entfernt ist, wird das Reaktionsgemisch wärmebehandelt, zweckmässig am Rückfluss erhitzt, wobei sich das entsprechende 2-substituierte 5-Nitroso-4,6-diamino-pyrimidin bildet. Unter saurem Milieu versteht man einen pH-Wert von unter 6,9 ; unter basischem Milieu versteht man einen pH-Wert von über 7,1.

Die Reaktion folgt der allgemeinen Formel

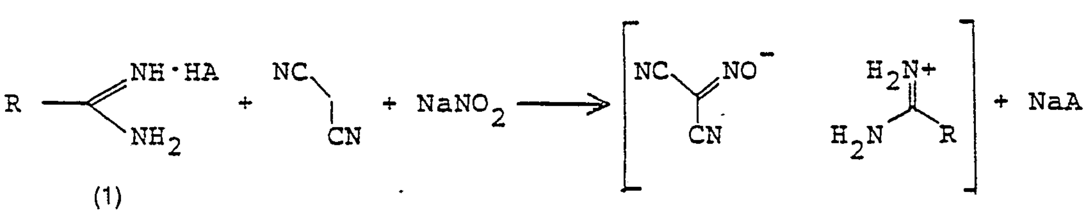

(1)

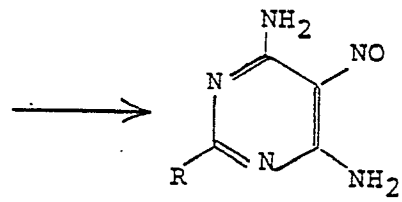

wobei A = Cl, 1/2 $SO_4$, $HSO_4$, $NO_3$, Acetat oder 1/3 $PO_4$ und R = Aryl, Alkyl, Alkylthio, Amino, mit 1 oder 2 $C_1$-$C_4$-Alkylgruppen substituiertes Amino oder Aralkyl bedeutet.

Beispiele für (1) sind Acetamidinhydrochlorid, Benzamidinhydrochlorid, S-Methylisothioharnstoffsulfat und Guanidin-hydrochlorid.

Nach speziellen Ausführungsformen der Erfindung hat der Substituent R in obigen Formeln vorzugsweise folgende Bedeutung : Als Aryl = Phenyl, als Alkyl = $C_1$-$C_4$-Alkyl, als Alkylthio = $C_1$-$C_4$-Alkylthio, als Arylalkyl = Phenyl-$C_1$-$C_4$-Alkyl.

Als Nitritsalz können die Alkali- oder Erdalkalimetallnitrite, vorzugsweise Natriumnitrit, verwendet werden.

Bezüglich der Mengenverhältnisse werden zweckmässig 0,1 bis 1,1 Mol Nitrit, vorzugsweise 1,01 Mol Nitrit, pro Mol Malonsäuredinitril angewendet.

Die Lösungsmittelmenge für die erste Reaktionsstufe ist nicht kritisch und beträgt zweckmässig 200 bis 2 000 ml pro Mol Malonsäuredinitril. Bevorzugt werden 300 bis 400 ml Lösungsmittel pro Mol Malonsäuredinitril eingesetzt.

In der zweiten Reaktionsstufe, mit Dimethylformamid oder einer Pyridinbase als Lösungsmittel, können pro Mol Malonsäuredinitril 100 bis 2 000 ml, bevorzugt 300 bis 500 ml, Lösungsmittel eingesetzt werden.

In einer vorzugsweisen Ausführungsform wird 5-Nitroso-2,4,6-triamino-pyrimidin so hergestellt, dass Malonsäuredinitril und Guanidin-hydrochlorid bei einem pH unter 6,9 in Gegenwart von Natriumnitril zum Guanidinsalz des Isonitroso-malonitrils umgesetzt und durch Kochen am Rückfluss in Dimethylformamid reagiert das Guanidinsalz des Isonitroso-malonitrils zum 5-Nitroso-2,4,6-triamino-pyrimidin umgewandelt wird.

Nach einer speziellen Ausführungsform des erfindungsgemäßen Verfahrens wird die Wärmebehandlung durch Erhitzen unter Rückfluß durchgeführt.

Nach der Isomerisierungs-Reaktion kann die rote Suspension von 5-Nitroso-2,4,6-triamino-pyrimidin in Dimethylformamid mit Wasser verdünnt, das Produkt durch Filtration oder Zentrifugieren abgetrennt und mit Wasser gewaschen werden. Für viele Umsetzungen kann das feuchte Produkt verwendet werden. Wenn nötig, kann es durch Erhitzen in der üblichen Weise getrocknet werden. Das 5-Nitroso-2,4,6-triamino-pyrimidin ist ein vielseitiges Zwischenprodukt, z. B. zur Herstellung von Medikamenten, wie Triamteren, Methothrexat, und zur Herstellung von Farbstoff-Komponenten, wie 2,4,5,6-Tetraaminopyrimidin.

## Beispiel 1

Zu einer Suspension von 66 g Malonitril und 96 g Guanidin-hydrochlorid in 200 g Wasser tropfte man bei Raumtemperatur eine Lösung von 70 g Natriumnitrit in 120 g Wasser hinzu, wobei durch Zugabe von Salzsäure der pH bei 4 gehalten wurde. Nach 4-stündiger Nachreaktion bei Raumtemperatur wurden 21 g Natriumcarbonat und 400 g Dimethylformamid zugegeben und das Wasser wurde bei vermindertem Druck abdestilliert. Anschliessend wurde das Reaktionsgemisch während 1 Stunde bei 140 °C erhitzt, wobei Isomerisierung zu 5-Nitroso-2,4,6-triaminopyrimidin stattfand. Nach Beendeter Reaktion wurden 400 ml Wasser zugegeben und das Produkt wurde abfiltriert und mit Wasser gewaschen.

Nach der Trocknung erhielt man 140 g reines himbeerrotes 5-Nitroso-2,4,6-triamino-pyrimidin ; Smp. über 340 °C.

Ausbeute : 91 %.

## Beispiel 2

Zu einer Suspension von 33 g Malonitril und 52 g Acetamidinhydrochlorid in 100 g Wasser tropfte man bei pH 4 und bei Raumtemperatur eine Lösung von 37,5 g Natriumnitrit in 60 g Wasser hinzu. Nach einer Nachreaktion von 4 Stunden wurde die Reaktionsmischung auf 0 °C gekühlt und das Produkt abfiltriert. Man erhielt das Acetamidinsalz von Isonitroso-malonitril mit dem Smp. 142 bis 143 °C (Zersetzung) in hoher Ausbeute (84 % isoliert).

## Beispiel 3

Zu einer Suspension von 13,2 g Malonitril und 32 g Benzamidinhydrochlorid in 25 g Wasser tropfte man bei pH 3 bis 5 und bei 20 °C eine Lösung von 14 g Natriumnitrit in 25 g Wasser hinzu. Nach einer Nachreaktion von 5 Stunden und Abkühlen auf 0 °C wurde das Reaktionsprodukt abgenutscht und

getrocknet. Man erhielt das Benzamidinsalz von Isonitroso-malonitril mit dem Smp. 150 °C (Zersetzung) in hoher Ausbeute (94 % isoliert).

Beispiel 4

Zu einer Suspension von 33 g Malonitril und 70 g S-Methylisothioharnstoff-sulfat in 100 ml Wasser tropfte man bei pH 4 und bei Raumtemperatur eine Lösung von 35 g Natriumnitrit in 60 ml Wasser hinzu. Nach einer Nachreaktion von 5 Stunden wurde das Reaktionsgemisch auf 4 °C gekühlt und abfiltriert.

Nach dem Trocknen erhielt man das S-Methylisothiouroniumsalz von Isonitroso-malonitril in sehr hoher Ausbeute (76 % isoliert), Smp. 123 bis 124 °C (Zersetzung).

Beispiel 5

Zu einer Suspension von 66 g Malonitril und 97 g Guanidinhydrochloridin 120 ml Wasser tropfte man bei pH 4 und bei Raumtemperatur eine Lösung von 70 g Natriumnitrit in 120 ml Wasser hinzu. Nach 4 Stunden Rühren wurde auf 0 °C gekühlt und abfiltriert.

Nach dem Trocknen im Vacuum erhielt man das Guanidinsalz von Isonitroso-malonitril in hoher Ausbeute (84 % isoliert), Smp. 160 bis 161 °C (Zersetzung).

Die weitere Umsetzung dieser Amidin-Salze des Isonitrosomalonitrils aus den Beispielen 2 bis 5 zu den entsprechenden 2-substituierten 5-Nitroso-4,6-diamino-pyrimidinen kann, wie in der Literatur E.C. Taylor et al, J. Am. Chem. Soc. 81, 2442 (1959) beschrieben, durchgeführt werden.

**Patentansprüche**

1. Verfahren zur Herstellung von 2-substituierten 5-Nitroso-4,6-diamino-pyrimidinen der allgemeinen Formel

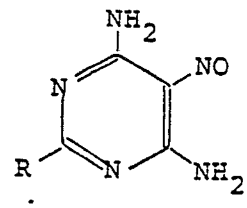

worin R = Aryl, Alkyl, Alkylthio, Amino, mit 1 oder 2 $C_1$-$C_4$-Alkylgruppen substituiertes Amino oder Arylalkyl bedeutet, bei dem Malonsäuredinitril mit einem Amidin oder Guanidin der allgemeinen Formel

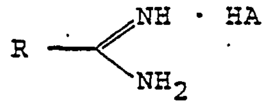

wobei A = Cl, 1/2 $SO_4$, $HSO_4$, $NO_3$, Acetat oder 1/3 $PO_4$ bedeutet und R die oben angegebene Bedeutung hat, in Wasser oder Alkohol in saurem Milieu in Gegenwart eines Nitritsalzes zum entsprechenden Amidin- bzw. Guanidinsalz des Isonitroso-malonitrils umgesetzt wird und dieses Salz dann in an sich bekannter Weise in Dimethylformamid oder einer Pyridinbase in basischem Milieu durch Wärmebehandlung ins entsprechende 2-substituierte 5-Nitroso-4,6-diamino-pyrimidin umgewandelt wird.

2. Verfahren nach Anspruch 1, dadurch gekennzeichnet, daß als Nitritsalz das Alkali- oder Erdalkalimetallnitrit angewendet wird.

3. Verfahren nach Anspruch 2, dadurch gekennzeichnet, daß als Nitritsalz Natriumnitrit angewendet wird.

4. Verfahren nach einem oder mehreren der Ansprüche 1 bis 3 zur Herstellung von 5-Nitroso-2,4,6-triamino-pyrimidin, dadurch gekennzeichnet, daß Malonsäuredinitril mit Guanidinhydrochlorid in Wasser bei einem pH unter 6,9 in Gegenwart von Natriumnitrit zum Guanidinsalz des Isonitroso-malonitrils und dieses Salz dann in basischem Milieu durch Kochen in einem Lösungsmittel am Rückfluss zum 5-Nitroso-2,4,6-triamino-pyrimidin umgesetzt wird.

**Claims**

1. A process for producing 2-substituted 5-nitroso-4,6-diamino pyrimidine of the general formula :

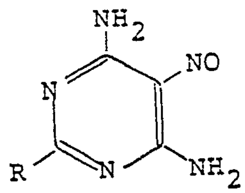

wherein R is aryl, alkyl, alkylthio, amino, amino substituted by one or two $C_1$ to $C_4$ alkyl groups, or arylalkyl, which comprises the steps of reacting malonic dinitrile with an amidine or guanidine of the general formula :

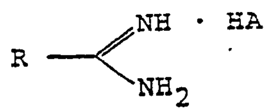

wherein A is Cl, 1/2 $SO_4$, $HSO_4$, $NO_3$, acetate, or 1/3 $PO_4$, and R has the meaning stated above, in water or alcohol in an acid milieu and in the presence of a nitrite salt to form the respective amidino or guanidino salt of isonitroso-malonitrile, and converting the salt thus obtained in a manner known per se by thermal treatment in dimethyl formamide or a pyridine base in a basic milieu to produce the respective 2-substituted 5-nitroso-4,6-diamino pyrimidine.

2. The method according to claim 1 wherein the nitrite salt is an alkali or alkaline earth metal nitrite.

3. The method according to claim 2 wherein the nitrite salt is sodium nitrite.

4. A method according to one or more of the claims 1 to 3 for producing 5-nitroso-2,4,6-triamino pyrimidine, characterized in that malonic dinitrile is reacted with guanidine hydrochloride in water at a pH below 6.9 in the presence of sodium nitrite to form the guanidine salt of isonitrosomalonitrile which is then reacted in a basic milieu by boiling at reflux in a solvent to produce 5-nitroso-2,4,6-triamino pyrimidine.

**Revendications**

1. Procédé pour la préparation de 5-nitroso-4,6-diamino-pyrimidines substituées en position 2 de formule générale

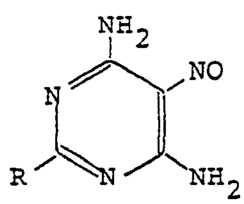

dans laquelle R = aryle, alcoyle, alcoylthio, amino, aminò substitué par 1 ou 2 groupes alcoyle en $C_1$-$C_4$ ou aralcoyle, selon lequel on fait réagir le dinitrile de l'acide malonique avec une amidine ou guanidine de formule générale

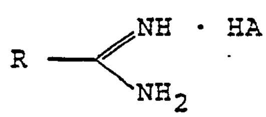

où A = Cl, 1/2 $SO_4$, $HSO_4$, $NO_3$, acétate ou 1/3 $PO_4$ et R a la signification indiquée plus haut, dans l'eau ou dans l'alcool en milieu acide en présence d'un sel de type nitrite pour donner le sel d'amidine ou de guanidine de l'isonitroso-malonitrile correspondant et en ce que l'on transforme ensuite ce sel de manière connue en soi dans le diméthylformamide ou dans une base pyridinique en milieu basique par traitement thermique en la 5-nitroso-4,6-diamino-pyrimidine substituée en position 2 correspondante.

2. Procédé selon la revendication 1, caractérisé en ce que l'on utilise en tant que sel de type nitrite, le nitrite d'un métal alcalin ou alcalino-terreux.

3. Procédé selon la revendication 2, caractérisé en ce que l'on utilise en tant que sel de type nitrite le nitrite de sodium.

4. Procédé selon l'une ou plusieurs des revendications 1 à 3 pour la préparation de la 5-nitroso-2,4,6-triamino-pyrimidine, caractérisé en ce que l'on fait réagir le dinitrile de l'acide malonique avec du chlorhydrate de guanidine dans l'eau à un pH inférieur à 6,9 en présence de nitrite de sodium pour donner le sel de guanidine de l'isonitroso-malonitrile et en ce qu'on transforme ensuite ce sel en milieu basique par ébullition dans un solvant au reflux en la 5-nitroso-2,4,6-triamino-pyrimidine.